Europäisches Patentamt

European Patent Office

Office européen des brevets

(19)

(11) Veröffentlichungsnummer: **0 688 873 A2**

# (12) EUROPÄISCHE PATENTANMELDUNG

(21) Anmeldenummer: **95108890.5**

(51) Int. Cl.6: **C12Q 1/68**

(22) Anmeldetag: **09.06.95**

(30) Priorität: **23.06.94 DE 4421901**

(43) Veröffentlichungstag der Anmeldung:
**27.12.95 Patentblatt 95/52**

(84) Benannte Vertragsstaaten:
**AT BE CH DE DK ES FR GB IT LI NL SE**

(71) Anmelder: **BAYER AG**

**D-51368 Leverkusen (DE)**

(72) Erfinder: **Springer, Wolfgang, Dr.**
**Katernberger Schulweg 31**
**D-42113 Wuppertal (DE)**
Erfinder: **Bremm, Klaus-Dieter, Dr.**
**Eberhardstr. 20**
**D-45661 Recklinghausen (DE)**
Erfinder: **Endermann, Rainer, Dr.**
**In den Birken 152a**
**D-42113 Wuppertal (DE)**

(54) **Ein DNA-Schnelltest zum Nachweis von chinolonresistenten Staphylococcus aureus Erregern in klinischem Probenmaterial**

(57) In der Erfindung werden Starteroligonukleotide (Primer), DNA-Sonden und Testverfahren zum schnellen Nachweis von chinolonresistenten Staphylococcus aureus Erregern in klinischem Probenmaterial beschrieben.

EP 0 688 873 A2

EP 0 688 873 A2

In der Erfindung werden Starteroligonukleotide (Primer), DNA-Sonden und Testverfahren zum schnellen Nachweis von chinolonresistenten Staphylococcus aureus Erregern in klinischem Probenmaterial beschrieben.

Fluorchinolone sind potente antibakterielle Agenzien zur Behandlung von schwierigen bakteriellen Infektionen. Auch von methicillinresistenten Staphylococcen verursachte Infektionen werden mit Fluorchinolonen z.B.Ciprofloxacin, behandelt.

Jedoch ähnlich wie bei den früheren Chinolonen, z.B. Nalidixinsäure, findet man immer häufiger auch Staphylococcus aureus Stämme, die resistent gegenüber Fluorchinolonen sind.

In vielen Kliniken bestehen inzwischen Probleme mit methicillinresistenten Staphylococcen, die auch resistent gegen Fluorchinolone geworden sind. Die Zunahme der Fluorchinolonresistenz ist von besonderer Bedeutung aufgrund der Tatsache,daß nur wenige antibakterielle Agenzien gegen methicillinresistente und chinolonresistente Staphylococcen-Infektionen wirksam sind.

Es ist deshalb besonders wichtig möglichst frühzeitig eine Therapie mit dem wirksamen Antibiotikum einzuleiten. Die Klassische Diagnostik der chinolonresistenten Staphylococcen ist zeit- und arbeitsaufwendig und erfordert die in vitro Kultivierung der Stämme auf Platten mit anschließender physiologischer Identifizierung mit Hilfe von Testsystemen wie z.B. "api staph" (BioMérieux) und Bestimmung der Antibiotikaresistenzen durch Reihenverdünnungsteste (MHK-Werte) oder Agardiffusionsteste (Hemmhofteste). Als Alternative bietet sich hier die Entwicklung eines Gentestes auf der Basis der Gyrase von QRSA an, der eine Schnelldiagnostik direkt im Probenmaterial ohne in vitro Kultivierung ermöglicht.

Ciprofloxacin ist ein 4-Chinolon-Antibiotikum, das die A2B2-Tetramer-Strukturdes Enzyms DNA-Gyrase hemmt. DNA-Gyrase bewirkt eine Verdrillung der DNA durch Katalyse von transienten Brüchen in der Doppelstrang-DNA.

Als molekulare Ursache der Chinolonresistenz von Staphylococcus aureus wurden Mutationen in der Gyrase A Untereinheit nachgewiesen (Sreedharan, S., M.Oram, B. Jensen, L.R. Peterson, L.M. Fisher, J. Bacteriol. 7260-7262, 1990). Eine weitere Ursache sind nor A Genmutationen, die einen membranassoziierten Transportmechanismus betreffen und so die Aufnahme bzw. interne Akkumulation des Antibiotikums verhindern (Yoshida, H., M. Bogaki, S., Nakamura, K.Ubukata, M.Konno, J.Bacteriol. 172, 6942-6949, 1990). Darüber hinaus wurde eine Mutation in einem weiteren Genlocus beschrieben, die über einen noch unbekannten Mechanismus Chinolonresistenz bewirkt (Trucksis, M., J.S. Wolfson, D.C.Hooper, J.Bacteriol. 173, 5854-5860, 1991).

Da die beschriebenen Punktmutationen des Serin 84 und -85 Codons im Gyrase A Gen von Staphylococcen mit Ciprofloxacin-Resistenz assoziiert sind, haben wir den Nukleotidbereich 2394 bis 2658, der diese Punktmutationen umfaßt, genauer untersucht.

In unseren Untersuchungen von 50 chinolonresistenten Staphylococcus aureus Isolaten mit unterschiedlichen MHK-Werten für Methicillin und Ciprofloxacin haben wir durch PCR-Sequenzierung mit magnetischen Beads modifiziert nach der Methode von Hultman T, Stahl, S., Hornes, E., Uhlen, M. Nucleic Acids Res. 17, 4937-4946, 1989, 4 Mutationstypen in der Gyrase A nachgewiesen (siehe Tabelle 1). Ein weiterer Mutationstyp basierend auf einer Punktmutation in Position 2540 bewirkt keinen Aminosäureaustausch auf Proteinebene und hat keinen Einfluß auf die Resistenzeigenschaft der Gyrase. Auf der Basis von drei Mutationen wurde ein punktmutationsspezifischer Amplifikationstest zum Nachweis von C/T, T/G und G/A Mutationen entwickelt.

Mit diesem Test werden alle für Ciproresistenz relevanten Gyrasemutationen bei Staphylococcus aureus erfaßt.

2

TABELLE 1

CIPRORESISTENZ-MUTATIONEN IN DER GYRASE A SEQUENZ
ANALYSIERTER SEQUENZBEREICH 2394 BIS 2658 DER GYRASE A
SEQUENZ VON STAPHYLOCOCCUS AUREUS

BEREICH 2526 BIS 2547 AUS DER GYRASE A SEQUENZ VON
STAPHYLOCOCCUS AUREUS

| Sequenz | Stamm | | Cipro-MHK $\mu g/ml$ |
|---|---|---|---|
| GGTGAC**TCA**TCT**ATT**TATGAAG | STAPH.AUREUS | LITERATUR | |
| | STAPH.AUREUS | NO.25532 | 0,5 |
| | STAPH.AUREUS | NO.25035 | <0,25 |
| | STAPH.AUREUS | NO.25085 | 0,25 |
| GGTGAC**TCA**TCT**ATC**TATGAAG | STAPH.AUREUS | NO.23 | 2,0 |
| | STAPH.AUREUS | NO.37 | 2,0 |
| | STAPH.AUREUS | NO.3 | 1,0 |
| | STAPH.AUREUS | NO.16 | 0,5 |
| | STAPH.AUREUS | NO.25470 | 0,5 |
| | STAPH.AUREUS | NO.25471 | 0,5 |
| | STAPH.AUREUS | NO.133 | <0,25 |
| GGTGAC**TTA**TCT**ATT**TATGAAG | STAPH.AUREUS | NO.25768 | >128 |
| | STAPH.AUREUS | NO.25911 | >128 |
| | STAPH.AUREUS | NO.25922 | >128 |
| | STAPH.AUREUS | NO.25936 | >128 |
| | STAPH.AUREUS | NO.25949 | >128 |
| | STAPH.AUREUS | NO.25920 | 128 |
| | STAPH.AUREUS | NO.30 | >64 |
| | STAPH.AUREUS | NO.25893 | 64 |
| | STAPH.AUREUS | NO.25894 | 64 |
| | STAPH.AUREUS | NO.25909 | 64 |
| | STAPH.AUREUS | NO.25913 | 64 |
| | STAPH.AUREUS | NO.25921 | 64 |
| | STAPH.AUREUS | NO.25948 | 64 |
| | STAPH.AUREUS | NO.44 | 32 |
| | STAPH.AUREUS | NO.64 | 32 |
| | STAPH.AUREUS | NO.25923 | 32 |
| | STAPH.AUREUS | NO.25912 | 16 |
| | STAPH.AUREUS | NO.25919 | 16 |
| | STAPH.AUREUS | NO.25924 | 16 |
| | STAPH.AUREUS | NO.25926 | 16 |
| | STAPH.AUREUS | NO.25928 | 16 |
| | STAPH.AUREUS | NO.25934 | 16 |
| | STAPH.AUREUS | NO.25906 | 8 |
| | STAPH.AUREUS | NO.25910 | 8 |
| GGTGAC**TTA**TCT**ATC**TATGAAG | STAPH.AUREUS | NO.25929 | 64 |
| | STAPH.AUREUS | NO.25703 | 32 |
| | STAPH.AUREUS | NO.24 | 16 |
| | STAPH.AUREUS | NO.25917 | 16 |
| | STAPH.AUREUS | NO.25925 | 16 |
| | STAPH.AUREUS | NO.25930 | 16 |
| | STAPH.AUREUS | NO.25932 | 16 |
| | STAPH.AUREUS | NO.25916 | 8 |
| | STAPH.AUREUS | NO.25927 | 8 |
| GGTGACTCATCTATTTAT**AAA**G | STAPH.AUREUS | NO.25915 | 64 |
| | STAPH.AUREUS | NO.25918 | 16 |
| GGT**GAT**TCATCTATTTAT**AAA**G | STAPH.AUREUS | NO.25914 | 32 |
| GGTGAC**GCA**TCTATTTATGAAG | STAPH.AUREUS | NO.25908 | 16 |

TCA > TTA     ATT > ATC     GAA > AAA

```
SERIN > LEUCIN        ISOLEUCIN  > ISOLEUCIN       GLUTAMIN > LYSIN

TCA    > GCA          GAC        > GAT
SERIN > ALANIN        ASPARAGIN  > ASPARAGIN
```

Mit diesem Gentest wurden 150 verschiedene chinolonresistente Staphylococcen (QRSA) (inclusive Stämme Tabelle 1) mit MHK-Werten zwischen 4 und 128 $\mu$g/ml (siehe auch Tabelle 2) analysiert. 95 % der Stämme weisen die Mutation C/T in Position 2533 auf. 4 Stämme (2,6%) weisen die Mutation T/G in Position 2532 auf und 3 Stämme (2%) weisen die Mutation G/A in Position 2544 auf. 5 Stämme (3,3%) weisen andere nicht in der Gyrase liegende Mutationen auf wie durch nachträgliche PCR-Sequenzierung dieser Stämme festgestellt wurde.

Damit ergibt sich für diesen Test auf der Basis der 3 nachzuweisenden Mutationen eine Detektionssensitivität von 97,7 %.

Tabelle 2  Genteste von QRSA

| Staphylococcus aureus | Methicillin MHK | Cipro-MHK | Gentest QRSA |
|---|---|---|---|
| 811 | >32 | 32 | C/T |
| 794 | >32 | 16 | C/T |
| 822 | >32 | 16 | - |
| 818 | >32 | >64 | C/T |
| 807 | 32 | 64 | C/T |
| 830 | 16 | >64 | C/T |
| 801 | >32 | 32 | C/T |
| 804 | >32 | >64 | C/T |
| 800 | >32 | 16 | G/A |
| 793 | 16 | >64 | C/T |
| 788 | >32 | 64 | C/T |
| 792 | >32 | >64 | C/T |
| 789 | >32 | 64 | C/T |
| 798 | 32 | >64 | C/T |
| 843 | >32 | >64 | C/T |
| 838 | 4 | 16 | T/G |
| 784 | >32 | 32 | - |
| 813 | 8 | >64 | C/T |
| 791 | >32 | 32 | C/T |
| 844 | 16 | >64 | C/T |
| 821 | 32 | 16 | - |
| 845 | >32 | 64 | C/T |
| 816 | >32 | 64 | C/T |
| 815 | >32 | 64 | C/T |
| 833 | >32 | 64 | - |

| Staphylococcus aureus | Methicillin MHK | Cipro-MHK | Gentest QRSA |
|---|---|---|---|
| 814 | >32 | 64 | C/T |
| 839 | 32 | 64 | C/T |
| 834 | >32 | 64 | C/T |
| 829 | >32 | 64 | C/T |
| 828 | >32 | >64 | C/T |
| 827 | >32 | 32 | C/T |
| 826 | >32 | 64 | C/T |
| 825 | >32 | 64 | C/T |
| 837 | >64 | >32 | C/T |
| 836 | >32 | >64 | C/T |
| 790 | >32 | 64 | - |
| 787 | >32 | >64 | C/T |
| 846 | 32 | 64 | C/T |
| 842 | >32 | 64 | C/T |
| 841 | >32 | 64 | C/T |
| 785 | >32 | >64 | C/T |
| 835 | >32 | >64 | C/T |
| 808 | >32 | >64 | C/T |
| 803 | >32 | >64 | C/T |
| 797 | >32 | >64 | C/T |
| 795 | 32 | 64 | C/T |
| 802 | >32 | >64 | C/T |
| 817 | >32 | 64 | C/T |

| Staphylococcus aureus | MethicillinMHK | Cipro-MHK | Gentest QRSA |
|---|---|---|---|
| 812 | >32 | >64 | C/T |
| 810 | >32 | >64 | C/T |
| 824 | >32 | >64 | C/T |
| 823 | 32 | 64 | C/T |
| 819 | >32 | >64 | C/T |
| | | | |
| Chinolonsensitive Stämme | Methicillin MHK | Cipro- MHK | Gentest QRSA |
| 25532 | 16 | 0,25 | - |
| 25035 | 4 | 0,25 | - |
| 25085 | 64 | 0,25 | - |
| 133 | 0,25 | 0,25 | - |
| 25470 | 32 | 0,5 | - |
| 25471 | 0,5 | 0,5 | - |
| 16 | 32 | 0,5 | - |

Die vorliegende Erfindung beschreibt spezifische Primer und Oligo-und Polynukleotidsonden und ihre Verwendung zum schnellen Nachweis von chinolonresistenten Staphylococcen direkt in klinischem Probenmaterial.

1. Die Primer wurden aus der Gensequenz des Gyrase A Gens durch chemische Synthese hergestellt.

Die bevorzugten Primer wurden aus dem Bereich ausgewählt

a) der spezifisch ist für den Mutationstyp C/T (Primer 1 SEQ ID No 1)

b) der spezifisch ist für den Mutationstyp T/G ( Primer 2 SEQ ID No 2)

c) der spezifisch ist für den Mutationstyp G/A ( Primer 3 SEQ ID No 3)

d) der spezifisch ist für chinolonresistente und sensitive Gyrase als Gegenstrang-Primer (Primer 4 SEQ ID No 4)

2. Die Oligonukleotidsonde wurde aus dem Bereich 2553-2588 durch chemische Synthese hergestellt( SEQ ID NO 5 ). Dieser Bereich wird von allen Primern amplifiziert und ist spezifisch für S.aureus.Die alternative Polynukleotidsonde (SEQ ID No 6) wurde durch PCR-Amplifikation des Nukleotidbereiches 2515 bis 3048 hergestellt.

3. Die genomische DNA von chinolonresistenten Stämmen wurde durch Nukleinsäureverfahren isoliert, die für Staphylococcen adaptiert wurden.

4. Die Amplifikation von Teilen des Gyrase A Gens wurde mit den spezifischen Primern 1-3 aus dem kodierenden Bereich,die am 3' Ende die Base der Resistenzmutation tragen ( T statt C, G statt T und A statt G) und einem spezifischen Primer 4 aus dem nicht kodierenden Strang des Gyrase A Gens, der als Gegenstrang-Primer für alle Amplifikationen eingesetzt wird, durchgeführt.

5. Die Amplifikation wurde mit Hilfe von bekannten Amplifikationstechniken, bevorzugt der PCR-DNA-Amplifikationsmethode (EP 200362) durchgeführt.

6. Der Nachweis des spezifischen Amplifikationsproduktes erfolgte durch

a) direkte Elektrophorese des Amplifikationsproduktes im Agarosegel und Anfärbung durch interkalierende Agenzien wie Ethidiumbromid

b) durch Fluoreszenzbestimmung des während der Amplifikation mit Fluoreszenznukleotiden oder fluoreszenzmarkierten Primern markierten Amplifikationsproduktes. Das Amplifikationsprodukt wird über zusätzlich eingebautes Biotin (Primer oder Nukleotid) separiert.

c) durch Hybridisierung des während der Amplifikation markierten Amplifikationsproduktes mit der obengenannten biotinylierten Oligonukieotidsonde. Der Hybridisierungskomplex wird mit Streptavidin gecoateten magnetischen Partikeln separiert.

d) Die Auswertung des gebildeten Hybridisierungskomplex als Maß für die Menge oder das Vorhandensein von chinolonresistenten Staphylococcen erfolgt durch einen Chemilumineszenztest mit Antidigoxigenin-Antikörpern, die mit alk. Phosphatase gekoppelt sind.

Die Auswertung kann bei Verwendung eines externen mitamplifizierten Staphylococcen-DNA-Standard semiquantitativ zur Ermittlung der Menge an chinolonresistenten Staphylococcen im klinischen Probenmaterial eingesetzt werden.

Die Gensonden-Diagnostik, insbesondere in Verbindung mit Amplifikationstechniken, ist eine schnelle,spezifische und hochempfindliche Methode, die eine Früherkennung der Erreger auf DNA/RNA Ebene ermöglicht.Die Technik kann direkt ohne in vitro Kultivierung im Untersuchungsmaterial durchgeführt werden. Sie basiert auf der DNA/RNA Hybridisierungstechnik d.h. der spezifischen in vitro Bindung von komplementärer Einzelstrang-Nukleinsäure unter Bildung von Watson-Crick-Basenpaaren. Die gebildeten DNA/DNA oder DNA/RNA Doppel-stränge werden auch als Hybride bezeichnet. Zur Detektion der spezifischen DNA oder RNA eines Erregers durch die Hybridisieningsreaktion werden komplementäre sequenzspezifische Gensonden verwendet.Diese Gensonden sind entweder kurze, chemisch synthetisierte Oligonukleotidsonden mit einer Länge von 10-50 Nukleotiden oder DNA/RNA Fragmente von 0,5-10kb,die durch rekombinante Gentechniken hergestellt wurden.

Die Gensonden können photochemisch (N.Dattagupta, P.M.M. Rae, E.D. Huguenel, E. Carlson, A. Lyga, J.S. Shapiro, J.P. Albarella, Analytical Biochem. 177, 85, 1989) oder enzymatisch durch nick Translation (Rigby, P.W.J. et al, J. Mol. Biol. 113, 237, 1977) oder Random Primed Techniken (Feinberg und Vogelstein, Anal. Biochem. 132, 6, 1983) mit einer radioaktiven oder nicht radioaktiven Markierung versehen werden. Geeignet sind hierfür Markierungen mit $^{32}$P NTPs oder nicht radioaktive Markierungen mit Digoxigenin-dUTP, Biotin-dUTP, Fluorescein-dUTP oder direkte Markierung mit Enzymen wie alk. Phosphatase oder Horseradish Peroxidase.

Für die spezifische Hybridisierung zwischen der nachzuweisenden Nukleinsäure des Erregers und der erregerspezifischen Gensonde werden die Nukleinsäuren zunächst durch Denaturierung (Hitze oder Alkalibehandlung) in Einzelstränge getrennt und dann unter stringenten Bedingungen, die durch Temperatur, Ionenstärke der Puffer und organische Lösungsmittel erreicht werden, ganz spezifisch miteinander hybridisiert. Bei geeigneten Hybridisierungsbedingungen bindet die Gensonde nur an komplementäre Sequenzen der nachzuweisenden DNA oder RNA. Diese Hybridisierungsreaktion kann in verschiedenen Testformaten z.B. als Festphasenhybridisierung an einen Träger wie z.B. Nitrozellulose gekoppelter Target-DNA oder Gensonde oder als Flüssighybridisierung durchgeführt werden. Die Auswertung (Read Out) erfolgt über die Markierung der Gensonde mit einem Reportermolekül wie oben aufgeführt oder wie in dem hier dargestellten Reversed Phase Hybridisierungssystem über die Target-DNA, die während der Amplifikation mit Digoxigenin-dUTP markiert wird und die Gensonde, die zur Bindung an magnetische Partikel mit Biotin markiert wird. Der Hybridisierungskomplex aus Target-DNA und markierter Gensonde wird nach Entfernen von nicht gebundener Gensonde über das verwendete Reportermolekül quantitativ bestimmt. Dieser Read Out kann direkt erfolgen bei Fluoreszenz-Markierung oder radioaktiver Markierung oder indirekt durch Enzymteste und immunologische Verfahren mit Antikörperkonjugaten, die Enzyme wie die alk. Phosphatase enthalten und dann eine Farbreaktion oder Chemilumineszenz-Reaktion ermöglichen.

Die Testsensitivität mit dieser Gensonden-Diagnostik liegt im Bereich von $10^5$ bis $10^6$ Keimen auf der Basis der Detektion von Einzelgenen.Eine Erhöhung der Testsensitivität kann durch die Kombination mit DNA oder RNA-Amplifikationstechniken wie der PCR (EP 200362), LCR (EP 320308),NASBA (EP 329822)-,Qß (PCT 87/06270) oder HAS-Technik (EP 427074) erreicht werden. Mit diesen Techniken kann bis zu einer $10^9$ fachen Multiplikation der nachzuweisenden DNA erzielt werden. Durch die Kombination von Amplifikation und Hybridisierung wird so die Detektion von einzelnen DNA-Molekülen möglich.

Da bei Infektionen im Blut Keimzahlen von $10^1$-$10^3$ Keimen/ml auftreten, bietet sich mit dieser Technologie die Möglichkeit einer frühzeitigen Erkennung von chinolonresistenten Infektionsverläufen.

Es wird darüber hinaus der Einsatz dieser Primer, Oligo- und Polynukleotidsonden und Testverfahren zum Nachweis von ciproresistenten Staphylococcen in klinischem Probenmaterial beschrieben. Ein weiterer Vorteil dieses Verfahrens ist die Möglichkeit einer semiquantitativen Bestimmung der Menge an chinolonresistenten Infektionskeimen in dem Probenmaterial.

### Auswahl und Synthese von Primern

Für die Auswahl von geeigneten spezifischen Primern wurde die Nukleotidsequenz des Gyrase A Gens von der in Tabelle 1 aufgeführten chinonolonresistenten Staphylococcus aureus Isolaten herangezogen. Auf der Basis dieser Mutationssequenzen und der Wildtyp-Nukleotidsequenz (E.E.C Margerrison, R. Hopewell, L.M. Fisher, J. Bacteriol. 1596-1603, 1992) wurden Primer ausgewahlt,die am 3' Ende die Base der Punktmutation tragen. Für den Mutationstyp C/T wurde der Primer SEQ ID No 1 synthetisiert. Für den Mutationstyp T/G wurde der Primer 2 SEQ ID No 2 synthetisiert Für den Mutationstyp G/A wurde der Primer SEQ ID No 3 synthetisiert. Für die Amplifikation der Mutation tragenden Sequenz wurde als Gegenstrang-Primer der Primer 4 SEQ ID No 4 der allen Primer-Sets gemeinsam ist (Primer 1 + 4, 2 + 4, 3 + 4) synthetisiert.

Die chemische Synthese der ausgewählten Primer erfolgte nach der Phosphoramiditmethode von S.L. Beaucage und M. Caruthers, Tetrahedron Letters, 22, 1859, 1981.

### Amplifikation von genomischer QRSA DNA

Für die Amplifikation von Teilen des Gyrase A Gens wurden die oben genannten Primer jeweils als Primerset 1 (Primer 1 + 4),Primerset 2 (Primer2 + 4) und Primerset 3 (Primer 3 + 4) zur spezifischen Amplifikation der 3 Mutationstypen der QRSA-Gyrasen eingesetzt. Sie ergeben mit der genomischen DNA von chinolonsensitiven Gyrasegenen kein im Agarosegel sichtbares Amplifikationsproduki. Die genomische DNA mit den 3 Primersets gibt jeweils für das die entsprechende Mutation tragende Gyrasegen eine starke Amplifikationsbande und zeigt so nicht nur die Chinolonresistenz sondern auch gleichzeitig die in der Gyrase vorhandene Punktmutation an.

Bei der PCR-Amplifikation kann neben den 4 dNTPs (Desoxyadenosintriphosphat, Desoxyguanosintriphosphat, Desoxycytidintriphosphat und Thymidintriphosphat) zusätzlich z.B. Digoxigenin-dUTP in das Amplifikationsprodukt eingebaut werden. Dadurch kann das Amplifikationsprodukt mit einem Antidigoxigenin-Antikörper, der z.B. alk. Phosphatase gekoppelt enthält über einen Chemilumineszenztest mit AMPPD als Substrat oder einem Farbstofftest mit Brom-Chlor-Indolylphosphat und Nitroblautetrazolium ausgewertet werden.

Alternativ besteht die Möglichkeit fluoreszenzmarkierte Nukleosidtriphosphate wie z.B. Fluoreszein-dUTP oder Cumarin-dUTPs in das Amplifikationsprodukt einzubauen und das Amplifikationsprodukt mit viel höherer Sensitivität als mit Ethidiumbromidanfärbung zu identifizieren. Bei der Verwendung von biotinylierten Primern besteht so die Möglichkeit das fluoreszenzmarkierte, biotinylierte Amplifikationsprodukt über Streptavidin gecoatete magnetische Partikel abzutrennen und im Fluoreszenzphotometer quantitativ zu bestimmen.

### Auswahl und Synthese der Qligonukleotidsonde

Die Auswahl der für die Amplifikationsprodukte der Primersets spezifische Oligonukleotidsonde erfolgte aus dem Bereich 2553-2588, der für alle 3 mutationsspezifischen Amplitikationsprodukte gemeinsam ist. Es wurde ein 36 mer mit der Sequenz SEQ ID No 5 synthetisiert,das spezifisch für die 3 Amplifikationsprodukte ist.

Die chemische Synthese erfolgte nach der Phosphoramiditmethode von S.L. Beaucage und M. Caruthers, Tetrahedron Letters, 22, 1859, 1981.

Mit der Oligonukleotidsonde besteht die Möglichkeit, die mutationsspezifischen Amplifikationsprodukte spezifisch zu hybridisieren. Das Verfahren, bei dem zunächst mit den mutationsspezifische Primern Teile des Gyrase A Gens amplifiziert werden und dann anschließend das Amplifikationsprodukt spezifisch mit der Oligonukleotidsonde hybridisiert, hat gegenüber der reinen Amplifikationsdiagnostik folgende Vorteile:

Bei direkter Auswertung des Amplifikationsproduktes im Gel liegt die Testsensitivität bei ca 1000 Keimen/ml Probenmaterial. In Kombination mit der Oligonukleotidsonden-Hybridisierung und einem Chemilumineszenz-Read Out gelingt dagegen ein Nachweis von 10 Keimen/ml Probenmaterial.

## Auswahl und Synthese einer alternativen Polynukleotidsonde

Alternativ zur Oligonukleotidsonde wurde eine Polynukleotidsonde von 534 Basen aus der Nukleotidsequenz 2515-3048, die allen mutationsspezifischen Amplifikationsprodukten gemeinsam ist, ausgewählt. Die Synthese der Polynukleotidsonde erfolgte durch PCR-Amplifikation mit dem Primer 1 (SEQ ID No 1) und dem Primer 4 (SEQ ID No 4). Während der Amplifikation wurde für den Chemilumineszenztest gleichzeitig Biotin-d-UTP in die amplifizierte Gensonde eingebaut.

## Detektion von QRSA

Die QRSA können direkt nach Amplifikation von Teilen des Gyrase A Gens durch die in der Erfindung beschriebenen 3 Primersets mit beliebigen Ahalyseverfähren bestimmt werden.

Eine mögliche Read Out Methode ist die Anfärbung des durch Agarosegelelektrophorese separierten Amplifikationsproduktes mit interkalierenden Agenzien wie Ethidiumbromid.

Eine andere Möglichkeit ist der Einbau von fluoreszenzmarkierten Nukleosidtriphosphaten in das Amplifikationsprodukt. Hierdurch wird eine deutliche Verbesserung der Testsensitivität erreicht.

Eine weitere Möglichkeit ist die Verwendung von fluoreszenzmarkierten Primern für die Amplifikation oder die Kombination von biotinylierten Primern mit Fluoreszenznukleotiden, so daß ein endständig biotinyliertes, fluoreszenzmarkiertes Amplifikationsprodukt entsteht, das an Streptavidin gekoppelte magnetische Partikel gebunden und separiert werden kann und die Fluoreszenz semiquantitativ bestimmt werden kann.

Die sensitivste Methode, die gleichzeitig auch eine semiquantitative Auswertung ermöglicht, ist die beschriebene Methode der Hybridisierung der Amplifikationsprodukte der 3 mutationsspezifischen Primersets mit der beschriebenen Oligonukleotidsonde. Diese Methode erlaubt darüber hinaus eine Quantifizierung der QRSA in klinischem Probenmaterial. Beim Einbau von z.B. Digoxigenin-dUTP während der Amplifikation und Verwendung einer biotinylierten Oligonukleotidsonde, läßt sich der Hybridisierungskomplex an Streptavidin gecoateten magnetischen Partikeln separieren und bei Verwendung von Antidigoxigenin-Antikörpern,die mit alk. Phosphatase gekoppelt sind, mit AMPPD als Substrat über Chemilumineszenz semiquantitativ auswerten. Werden gleichzeitig Proben mit definierten Zellzählen als Zellstandard mitamplifiziert und im Chemilumineszenztest ausgewertet so kann aus den Chemilumineszenzsignalen des klinischen Probenmaterials die Keimzahl semiquantitativ bestimmt werden.

## Beispiel 1

## Synthese von Starteroligonukleotiden (Primer)

Die chemische Synthese der ausgewählten Starteroligonukleotide (Primer) erfolgte nach der Phosphoramiditmethode von S.L. Beaucage und M. Caruthers, Tetrahedron Letters, 22, 1859, 1981. Folgende Nukleotidsequenzen wurden synthetisiert:

PCR-Primer 1 spezifisch für Mutation C/T: SEQ ID No 1
PCR-Primer 2 spezifisch für Mutation C/G: SEQ ID No 2
PCR-Primer 3 spezifisch für Mutation G/A: SEQ ID No 3
PCR-Primer 4 Gegenstrangprimer für alle Mutationen: SEQ ID No 4

### Beispiel 2

### Synthese und Auswahl der Oligonukleotidsonde für ORSA

Die Oligonukleotidsonde wurde aus dem Nukleotidbereich 2553-2588, der in allen mutationsspezisichen Amplifikationsprodukten enthalten ist und spezifisch für die Gyrase A von Staphylococcus aureus ist, ausgewählt.

Die chemische Synthese der ausgewählten Oligonukleotidsonde erfolgte nach der Phosphoramiditmethode von S.L. Beaucage und M. Caruthers, Tetrahedron Letters, 22, 1859, 1981. Folgende Nukleotidsequenz wurde synthetisiert: GTACGTATGGCTCAAGATTTCAGTTATCGTTATCCG 36 mer Oligonukleotid aus Nukleotidbereich 2553-2588 SEQ ID No 5.

Die Oligonukleotidsonde wurde nach der Methode von Bollum, The enzymes, Boyer ed, Vol 10, p 145 Academic Press New York, am 3' Ende markiert. Die Endgruppenmarkierung wurde nicht radioaktiv mit Biotin-dUTP vorgenommen. (Chang,L.M.S.,Bollum T.J., J.Biol.Chem.246,909,1971).

In einem 50$\mu$l Ansatz mit 10$\mu$l Reaktionspuffer (Kaliumkakodylat 1mol/l; Tris/HCl 125mmol/l; Rinderserumalbumin 1,25mg/ml; pH6,6;25 °C) 1-2$\mu$g Oligonukleotid, 25 Einheiten Terminale Transferase, CoCl$_2$ 2,5mmol/l und 1$\mu$l Biotin-d-UTP (1mmol/L) werden nach 60 Minuten bei 37 °C ca. 50% 3' Endmarkierung erreicht.

### Beispiel 3

### Synthese und Auswahl einer Polynukleotidsonde für ORSA

Die Polynukleotidsonde wurde aus dem Nukleotidbereich 2515 bis 3048, der in allen mutationsspezifischen Amplifikationsprodukten enthalten ist und spezifisch ist für die Gyrase A von S.aureus, ausgewählt.

Die Synthese erfolgte durch PCR-Amplifikation mit dem Primer 1 (SEQ ID No 1) und dem Primer 4 ( SEQ ID No 4) nach Beispiel 4. Für den Chemilumineszenztest nach Beispiel 6 wurde während der Amplifikation Biotin-d-UTP in die amplifizierte Gensonde eingebaut.

### Beispiel 4

### ORSA spezifische DNA- Amplifikation mit der PCR

Die Amplifikation der Target-DNA wurde nach der Polymerase Chain Reaktion (EP 200362;201184) durchgeführt.

Zur PCR-Reaktion wurden eingesetzt: 100 pg genomische DNA von chinolonresistenten Staphylococcen, 0,17 $\mu$mol Primer 1-4 SEQ ID No 1-4, 2,5 Units Taq-Polymerase von Cetus/Perkin-Elmer sowie jeweils 200 $\mu$mol/l dNTPS in insgesamt 100 $\mu$l PCR-Puffer (50 mM KCl, 10 mM Tris/HCl pH 8,3, 2 mM MgCl$_2$, und 0,01 % Gelatine. Die Amplifikation wurde in einem PCR-Prozessor der Firma Cetus/Perkin-Elmer durchgeführt. Es wurden insgesamt 3 PCR-Ansätze mit Primerset 1 (Primer1 + 4) Primerset 2 (Primer 2 + 4) und Primerset 3 (3 + 4) spezifisch für die drei Gyrasemutationen angesetzt.

Für den Chemilumineszenztest nach Beispiel 6 wurde in der PCR zusätzlich 0,15 $\mu$mol Digoxigenin-d-UTP zur Markierung des PCR-Produktes eingesetzt.

Mit den Ansätzen wurde zunächst eine Initialschmelzung der DNA 2 Minuten, 30 Sek. bei 95 °C durchgeführt, dann pro Zyklus 1 Min. bei 95 °C die DNA denaturiert, 1 Minute bei 58 °C das Primer-Annealing und 1 Minute bei 72 °C die Primer-Extension durchgeführt. Nach 25 Zyklen wurden die Ansätze direkt bei 4 °C gekühlt.

### Beispiel 5

### Direkte Auswertung des Amplifikationsproduktes

Zur direkten Auswertung des DNA- Amplifikationsproduktes wurden nach der Amplifikation interkalierende Agenzien wie z.B. Ethidiumbromid eingesetzt oder während der Amplifikation Fluoreszenz-Nukleotidtriphosphate wie z.B: Fluoreszein-dUTP/ oder Hydroxy-Cumarin-dUTP eingebaut. Es können auch BiotindUTP oder Digoxigenin-dUTP eingesetzt werden und mit Antikörper gekoppelter alk. Phosphatase ein Farbstoff-Read Out durchgeführt werden. Auch können bei geringerer Sensitivität entsprechend floureszenzmarkierte Primer verwendet werden.

Das Amplifikationsprodukt wurde auf ein 0.8 %iges Agarosegel aufgetragen und 30 Minuten bei 100mA elektrophoretisiert. Die Fluoreszenz-Signale von chinolonsensitiven und chinolonresistenten Staphylococcen wurden unter einem UV-Transilluminator direkt ausgewertet.

**Beispiel 6**

**Chemilumineszenz-Gensondentest von DNA-Amplifikationsprodukten**

Durch die Kombination von geeigneten Target-Amplifikationsmethoden wie der Polymerase Chain Reaction (PCR) (EP200362), LCR (EP320308) und der Gensondentechnik wird eine signifikante Sensitivitätssteigerung gegenüber den herkömmlichen Gensonden-Read Out Methoden erzielt.

Die Flüssighybridisierungsteste wurden als Reversed Phase Teste mit 100ng 3'-biotinylierten Gensonden und amplifizierter DNA nach Beispiel 4 in einem Volumen von 50$\mu$l durchgeführt.

Nach 10 minütigem Erhitzen auf 100° C und anschließendem Abkühlen auf 0° C wurden 50$\mu$l 2x Hybridisierungsmix (50ml 20XSSC,1g Blocking Reagenz (Boehringer), 2 ml 10%iges Lauroylsarcosin, 200$\mu$l 20%iges SDS ad 100 ml bidest $H_2O$) zugegeben und 5-10 Minuten bei 37° C hybridisiert (Oligonukleotidsonde). Die magnetischen Beads wurden mit 1x Hybridisiermix vorbehandelt und nach dem Separieren über einen Magneten die Flüssigkeit abpipettiert, der Hybridisierungsansatz und 100$\mu$l 1x Hybridsiermix zugegeben und 5-10 Minuten bei Raumtemperatur unter schwacher Bewegung inkubiert. Der gekoppelte Hybridisierungskomplex wurde mit den Beads separiert, die Restflüssigkeit abpipettiert und einmal mit Puffer B (0,1 SSC;0,1%SDS) 1x gewaschen.

Anschließend wurde die Blocking Reaktion und Antikörper-Reaktion zum Nachweis der Hybridisierung über Chemilumineszenz durchgeführt. Die mit DNA beladenen Beads wurden 1x mit 500$\mu$l Puffer 2 (0,1M Maleinsäure; 0,15M NaCl pH7,5;1% Blocking Reagenz (Boehringer) zugegeben. Nach 5 Minuten Inkubation bei Raumtemperatur wurde separiert, abpipettiert und 250 $\mu$l Antikörperkonjugat-Lösung (AK 1:2500 in Puffer 2) zugeben und 10 Minuten bei Raumtemperatur inkubiert, dann separiert, abpipettiert und mit 500$\mu$l Waschpuffer behandelt 2x 30 Sekunden, 1x 2 Minuten bei schwacher Bewegung. Es wurde dann mit 100 $\mu$l Detektionslösung mit AMPPD 1:100 in Puffer 3 10 Minuten bei 37°C im Wasserbad inkubiert, dann die Chemilumineszenz im Lumineszenz-Photometer bei 477 nm (Lumacounter von Lumac) gemessen.

Mit diesem Test können DNA-Mengen entsprechend $10^6$ bis $10^1$ Staphylococcus Keime detektiert werden.Die gleiche Detektionsgrenze von 10 Keimen pro ml Blut wurde auch nach Zugabe von unspezifischer Blut-DNA erreicht.Blut-DNA alleine gibt nur geringe Background-Signale im Hybridisierungstest.

**Beispiel 7**

**Isolierung von Staphylococcen-Nukleinsäure**

Das mit Staphylococcen infizierte Probenmaterial, z.B. infiziertes Blut, wurde bei 8000 UpM 5-10 Minuten zentrifugiert, der Überstand abpipettiert und verworfen Das Sediment (ca 180-200$\mu$l) wurde mit 50$\mu$l TE mit 15 %iger Saccharose versetzt, 20$\mu$l Lysozym + Lysostaphin (10 + 5mg/ml in bidest.H2O) zugegeben und 15 Minuten bei 37°C inkubiert. Die weitere Aufarbeitung erfolgte mit einem Qiamp-DNA-Kit der Firma Diagen. Nach Zugabe von 25$\mu$l Proteinase K und 235$\mu$l AL Puffer wurde gemischt und 10 Minuten bei 70°C behandelt. Dann wurde in Eis abgekühlt und 240 $\mu$l 100%iges Ethanol zugegeben und nach kurzem Mischen auf die Qiagen-Säule aufgetragen. Anschließend wurde die Säule 1 Minute bei 8000UpM zentrifugiert, das Eluat verworfen .Es wurde 700$\mu$l AW Wasch-Puffer aufgetragen und nochmals 1 Minute bei 8000 UpM zentrifugiert. Anschließend wurde nochmals mit der gleichen Menge AW Puffer gewaschen und 1 Minute bei 8000 UpM und 10 Sekunden bei 14 000 UpM zentrifugiert und das Eluat verworfen. Es wurden dann 200 $\mu$l bidest $H_2O$ auf die Säule aufgetragen und 1 Minute bei 8000 UpM zentrifugiert. Das Eluat enthält die für die Amplifikation einsetzbare gereinigte Nukleinsäurelösung.

**Beispiel 8**

**Nachweis von QRSA in klinischem Probenmaterial**

Die QRSA DNA wurde aus dem Klinischen Probenmaterial nach der in Beispiel 7 beschriebenen Methode isoliert. Das Staphylococcen-DNA-Lysat wurde dann mit Hilfe geeigneter Amplifikationsmethoden wie im Beispiel 4 beschrieben mit QRSA spezifischen Oligonukleotid-Primern amplifiziert. Die amplifizierte Nukleinsäure wurde mit der Oligonukleotidsonde SEQ ID No 5 hybridisiert und der unter stringenten

12

Bedingungen sich ausbildende spezifische Hybridisierungskomplex aus amplifizierter Staphyloccocen Nukleinsäure und Gensonden-DNA wurde mit magnetischen Partikeln der Firma Dynal separiert und wie im Beispiel 6 durch Chemilumineszenz-Read Out quantitativ bestimmt.

In den mit QRSA infizierten Blutlysaten wurden Gyrase A Gen-spezifische Hybridisierungssignale noch in einer Konzentration von $10^1$ Keimen nachgewiesen.

## Beispiel 9

### Quantitativer Nachweis von QRSA

QRSA wurden wie im Beispiel 7 aus klinischem Probenmaterial, z.B. Blut, isoliert und nach Amplifikation wie in den Beispielen 4 und 6 beschrieben ein Chemilumineszenztest durchgeführt. Neben den klinischen Proben wurden Proben mit QRSA DNA entsprechend den Zellzahlen von $10^6$ bis 10 Zellen in 500ng Blut-DNA parallel amplifiziert und im Chemilumineszenztest analysiert. Die Chemilumineszenzsignale des Zellstandards von $10^6$ bis 10 QRSA im Blut wurden mit den parallel analysierten Testproben mit $10^3$ und $10^2$ Keimen, die im 3-Primerset-Test nachgewiesen worden waren, verglichen. Die Chemilumineszenzsignale aus dem Zellzahl-DNA-Standard ($10^3$ und $10^2$) stimmen sehr gut mit den Chemilumineszenzsignalen der Testproben mit $10^3$ und $10^2$ Keimen überein und zeigen, daß der Chemilumineszenztest zur semiquantitativen Messung der QRSA z.B. im Blut eingesetzt werden kann.

## Beschreibung der Abbildungen

### Abbildung 1

Direkte Auswertung des Amplifikationstestes. Die DNA von 3 chinolonresistenten S.aureus Erregern und eines chinolonsensitiven Wildstammes,entsprechend $10^3$ Zellen, wurde mit den punktmutationspezifischen Primersets 1 (Primer SEQ ID No 1 und SEQ ID No 4) Primerset 2 (Primer SEQ ID No 2 und SEQ ID No 4) und Primerset 3 (Primer SEQ ID No 3 und SEQ ID No 4) amplifiziert und dann durch Agarosegelelektrophorese analysiert. Eine Bande im Ethidiumbromid angefärbten Agarosegel ist nur sichtbar, wenn der resistente Stamm die entsprechende Punktmutation im Gyrase A Gen enthält. Der chinolonsensitive S.aureus Wildtyp-(WT25035) ergibt keine Amplifikationsbande mit den 3 Primersets, weil keine Punktmutation in der Wildtyp-Gyrase vorliegt. Der S.aureus Stamm 25768 hat eine C/T-Mutation, der Stamm 25908 eine T/G-Mutation und der Stamm 25918 eine G/A-Mutation.

### Abbildung 2 A und B

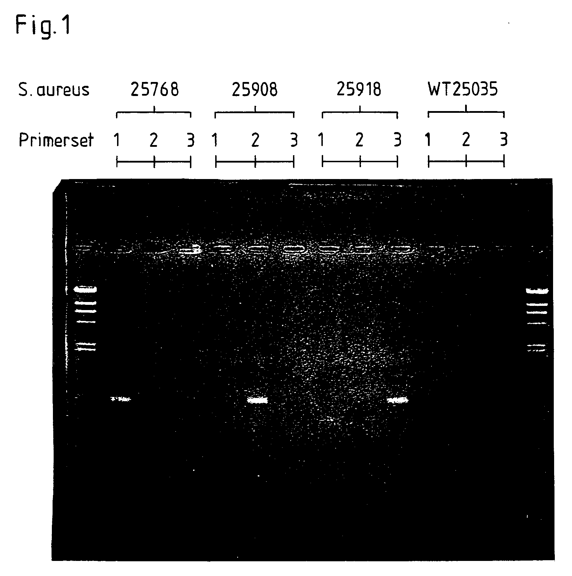

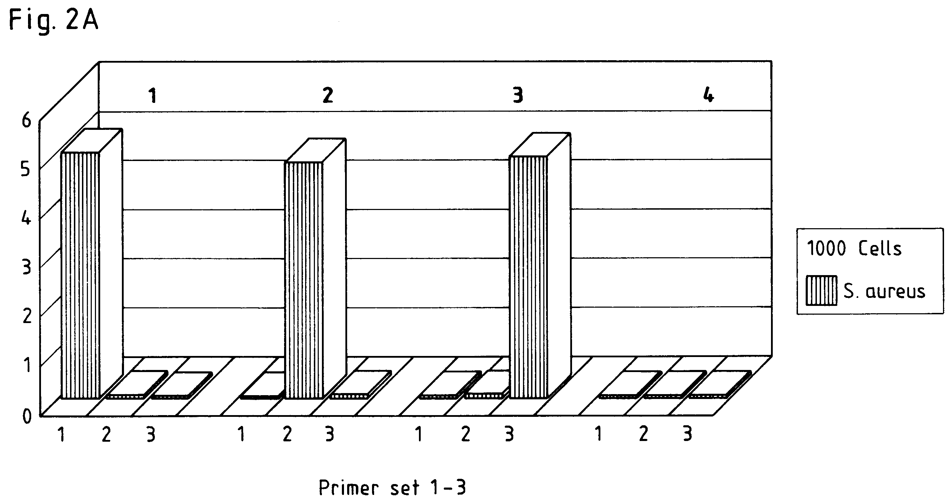

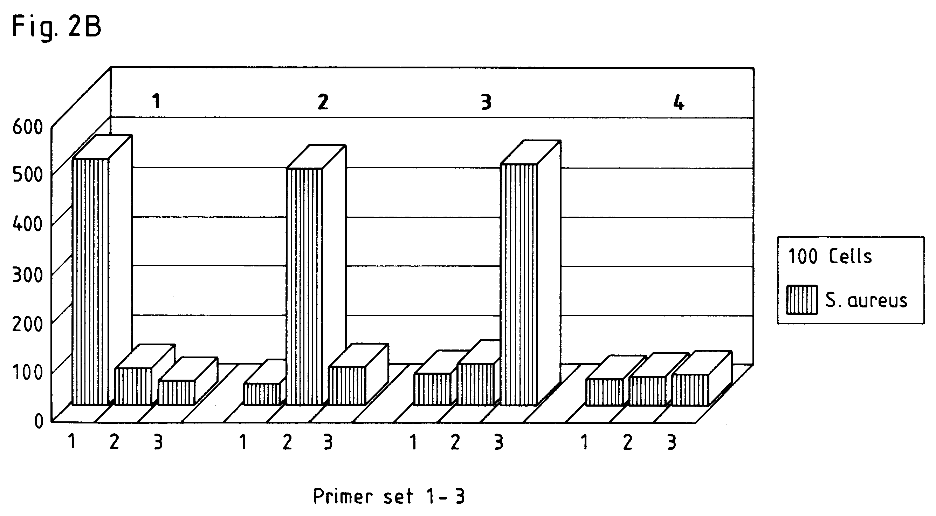

Die DNA von 3 chinolonresistenten S.aureus Erregern, 25768 (1), 25908 (2), 25918(3), entsprechend ca 1000 Zellen (Fig 2A) oder ca 100 Zellen (Fig.2 B) und einem chinolonsensitiven S.aureus Wildtyp (25035 (4)) wurde mit den punktmutationsspezifischen Primersets 1-3 (SEQ ID No 1-4) amplifiziert und dabei Digoxigenin-d-UTP in das Amplifikationsprodukt eingebaut. Die Amplifikationsprodukte wurden mit einer biotinylierten Oligonukleotidsonde (SEQ ID No 5) hybridisiert und der Hybridisierungskomplex mit Streptavidin gecoateten magnetischen Partikeln abgetrennt. Die Bildung des Amplifikationsproduktes wurde über das Chemilumineszenzsignal nach Beispiel 6 gemessen. Ein Chemilumineszenzsignal entsteht nur dann, wenn der resistente Stamm die entsprechende Punktmutation im Gyrase A Gen enthält und deshalb durch das entsprechende Primerpaar 1, 2 oder 3 amplifiziert wurde. Der chinolonsensitive S.aureus Stamm 25035 ergibt kein Chemilumineszenzsignal mit den 3 Primersets, weil keine Punktmutation in der Wildtyp-Gyrase vorliegt. Der S.aureus Stamm 25768 ist aufgrund einer C/T-Mutation resistent, der Stamm 25908 aufgrund einer T/G-Mutation und der Stamm 25918 aufgrund einer G/A-Mutation wie aus der Amplifikation mit den entsprechenden Primersets ersichtlich.

EP 0 688 873 A2

**Patentansprüche**

1. Starteroligonukleotide (Primer) für die Amplifikation und den spezifischen Nachweis von C/T Punktmutationen in Position 2533 der Nuleotidsequenz der Gyrase A, dadurch gekennzeichnet, daß sie die Nukleotidsequenz wie im Sequenzprotokoll SEQ ID No 1 und 4 enthalten.

2. Starteroligonukleotide (Primer) für die Amplifikation und den spezifischen Nachweis von T/G Punktmutationen in Position 2532 der Nuleotidsequenz der Gyrase A, dadurch gekennzeichnet, daß sie die Nukleotidsequenz wie im Sequenzprotokoll SEQ ID No 2 und 4 enthalten.

3. Starteroligonukleotide (Primer) für die Amplifikation und den spezifischen Nachweis von G/A Punktmutationen in Position 2544 der Nukleotidsequenz der Gyrase A, dadurch gekennzeichnet, daß sie die Nukleotidsequenz wie im Sequenzprotokoll SEQ ID No 3 und 4 enthalten.

4. Eine Qligonukleotidsonde, die mit DNA oder RNA von chinolonresistenten Staphylococcen hybridisiert, dadurch gekennzeichnet, daß sie die Nukleotidsequenz wie im Sequenzprotokoll SEQ ID No 5 enthält oder markierte Sequenzen davon.

5. Eine Polynukleotidsonde, die mit DNA oder RNA von chinolonresistenten Staphylococcen hybridisiert, dadurch gekennzeichnet, daß sie 534 Basen oder Teile davon enthält und die Nukleotidsequenz wie im Sequenzprotokoll SEQ ID No 6 oder Teile davon enthält oder markierte Sequenzen davon.

6. Verfahren zum Nachweis von chinolonresistenten Staphylococcus aureus Keimen in Probenmaterial, dadurch gekennzeichnet, daß
   a) die Nukleinsäure von chinolonresistenten Staphylococcus aureus Keimen nach Amplifikation mit mutationsspezifischen Primersets nach Anspruch 1-3 direkt ohne weitere Hybridisierung analysiert wird oder zur Reaktion mit Gensonden eingesetzt wird.
   b) Oligonukleotid- oder Polynukleotidsonden nach Anspruch 4 oder 5 zur spezifischen Hybridisierung und Detektion von chinolonresistenter Nukleinsäure von Staphyloccocus aureus eingesetzt werden.
   c) Oligo-und Polynukleotidsonden mit Hilfe von bekannten Methoden mit Reportermolekülen markiert werden.
   d) Der Hybridisierungskomplex über die Markierung der amplifizierten Target-DNA ein direktes Maß für den Nachweis und die Menge an chinolonresistenten Staphylococcen liefert.

7. Ein Verfahren nach Anspruch 6 dadurch gekennzeichnet, daß für die Amplifikation von Teilen des Gyrase A Gens die Primer nach Anspruch 1-3 eingesetzt werden.

8. Ein Verfahren nach Anspruch 6, dadurch gekennzeichnet, daß eine Oligonukleotidsonde nach Anspruch 4 verwendet wird.

9. Ein Verfahren nach Anspruch 6, dadurch gekennzeichnet, daß eine Polynukleotidsonde nach Anspruch 5 verwendet wird.

10. Testkit, enthaltend eine oder mehrere der Oligonukleotide nach Ansprüchen 1 bis 5 sowie Puffer und andere Lösungen zur praktischen Durchführung einer Analyse auf Chinolonresistenz in klinischem Probenmaterial.

# Fig.1

# Fig. 2A

Primer set 1-3

EP 0 688 873 A2

Fig. 2B

Primer set 1- 3

100 Cells

S. aureus

EP 0 688 873 A2

SEQUENZPROTOKOLL

(1) ALGEMEINE INFORMATION:

    (i) ANMELDER:
       (A) NAME: Bayer AG
       (B) STRASSE: Bayerwerk
       (C) ORT: Leverkusen
       (E) LAND: Deutschland
       (F) POSTLEITZAHL: D-51368
       (G) TELEPHON: 0214/3061455
       (H) TELEFAX: 0214/303482

    (ii) ANMELDETITEL: Ein DNA-Schnelltest zum Nachweis von
       chinolonresistenten Staphylococcus aureus Erregern in
       klinischem Probenmaterial

   (iii) ANZAHL DER SEQUENZEN: 13

    (iv) COMPUTER-LESBARE FORM:
       (A) DATENTRÄGER: Floppy disk
       (B) COMPUTER: IBM PC compatible
       (C) BETRIEBSSYSTEM: PC-DOS/MS-DOS
       (D) SOFTWARE: PatentIn Release #1.0, Version #1.25 (EPA)

(2) INFORMATION ZU SEQ ID NO: 1:

    (i) SEQUENZ CHARAKTERISTIKA:
       (A) LÄNGE: 19 Basenpaare
       (B) ART: Nukleinsäure
       (C) STRANGFORM: Einzel
       (D) TOPOLOGIE: linear

    (ii) ART DES MOLEKÜLS: DNS (genomisch)

   (iii) HYPOTHETISCH: NEIN

   (iii) ANTISENSE: NEIN

    (vi) URSPRÜNLICHE HERKUNFT:
       (A) ORGANISMUS: Primer
       (C) INDIVIDUUM ISOLAT: synthetisch

    (xi) SEQUENZBESCHREIBUNG: SEQ ID NO: 1:

ATCACCCTCA TGGTGACTT                                19

(2) INFORMATION ZU SEQ ID NO: 2:

    (i) SEQUENZ CHARAKTERISTIKA:
       (A) LÄNGE: 18 Basenpaare
       (B) ART: Nukleinsäure
       (C) STRANGFORM: Einzel
       (D) TOPOLOGIE: linear

    (ii) ART DES MOLEKÜLS: DNS (genomisch)

   (iii) HYPOTHETISCH: NEIN

   (iii) ANTISENSE: NEIN

    (vi) URSPRÜNLICHE HERKUNFT:
       (A) ORGANISMUS: Primer
       (C) INDIVIDUUM ISOLAT: synthetisch

    (xi) SEQUENZBESCHREIBUNG: SEQ ID NO: 2:

ATCACCCTCA TGGTGACG                                18

(2) INFORMATION ZU SEQ ID NO: 3:

14

```
      (i) SEQUENZ CHARAKTERISTIKA:
          (A) LÄNGE: 21 Basenpaare
          (B) ART: Nukleinsäure
          (C) STRANGFORM: Einzel
          (D) TOPOLOGIE: linear

     (ii) ART DES MOLEKÜLS: DNS (genomisch)

    (iii) HYPOTHETISCH: NEIN

    (iii) ANTISENSE: NEIN

     (vi) URSPRÜNLICHE HERKUNFT:
          (A) ORGANISMUS: Primer
          (C) INDIVIDUUM ISOLAT: synthetisch


     (xi) SEQUENZBESCHREIBUNG: SEQ ID NO: 3:

ATGGTGACTC ATCTATTTAT A                                     21

(2) INFORMATION ZU SEQ ID NO: 4:

      (i) SEQUENZ CHARAKTERISTIKA:
          (A) LÄNGE: 18 Basenpaare
          (B) ART: Nukleinsäure
          (C) STRANGFORM: Einzel
          (D) TOPOLOGIE: linear

     (ii) ART DES MOLEKÜLS: DNS (genomisch)

    (iii) HYPOTHETISCH: NEIN

    (iii) ANTISENSE: NEIN

     (vi) URSPRÜNLICHE HERKUNFT:
          (A) ORGANISMUS: Primer
          (C) INDIVIDUUM ISOLAT: synthetisch


     (xi) SEQUENZBESCHREIBUNG: SEQ ID NO: 4:

GTCCGCCTCC ACGTTCTT                                         18

(2) INFORMATION ZU SEQ ID NO: 5:

      (i) SEQUENZ CHARAKTERISTIKA:
          (A) LÄNGE: 36 Basenpaare
          (B) ART: Nukleinsäure
          (C) STRANGFORM: Einzel
          (D) TOPOLOGIE: linear

     (ii) ART DES MOLEKÜLS: DNS (genomisch)

    (iii) HYPOTHETISCH: NEIN

    (iii) ANTISENSE: NEIN

     (vi) URSPRÜNLICHE HERKUNFT:
          (A) ORGANISMUS: Primer
          (C) INDIVIDUUM ISOLAT: synthetisch


     (xi) SEQUENZBESCHREIBUNG: SEQ ID NO: 5:

GTACGTATGG CTCAAGATTT CAGTTATCGT TATCCG                     36

(2) INFORMATION ZU SEQ ID NO: 6:

      (i) SEQUENZ CHARAKTERISTIKA:
          (A) LÄNGE: 534 Basenpaare
          (B) ART: Nukleinsäure
          (C) STRANGFORM: Einzel
          (D) TOPOLOGIE: linear

     (ii) ART DES MOLEKÜLS: DNS (genomisch)

    (iii) HYPOTHETISCH: NEIN
```

(iii) ANTISENSE: NEIN

(vi) URSPRÜNLICHE HERKUNFT:
        (A) ORGANISMUS: Sonde
        (C) INDIVIDUUM ISOLAT: synthetisch

(xi) SEQUENZBESCHREIBUNG: SEQ ID NO: 6:

```
ATCACCCTCA TGGTGACTCA TCTATTTATG AAGCAATGGT ACGTATGGCT CAAGATTTCA      60
GTTATCGTTA TCCGCTTGTT GATGGCCAAG GTAACTTTGG TTCAATGGAT GGAGATGGCG     120
CAGCAGCAAT GCGTTATACT GAAGCGCGTA TGACTAAAAT CACACTTGAA CTGTTACGTG     180
ATATTAATAA AGATACAATA GATTTTATCG ATAACTATGA TGGTAATGAA AGAGAGCCGT     240
CAGTCTTACC TGCTCGATTC CCTAACTTGT TAGCCAATGG TGCATCAGGT ATCGCGGTAG     300
GTATGGCAAC GAATATTCCA CCACATAACT TAACAGAATT AATCAATGGT GTACTTAGCT     360
TAAGTAAGAA CCCTGATATT TCAATTGCTG AGTTAATGGA GGATATTGAA GGTCCTGATT     420
TCCCAACTGC TGGACTTATT TTAGGTAAGA GTGGTATTAG ACGTGCATAT GAAACAGGTC     480
GTGGTTCAAT TCAAATGCGT TCTCGTGCAG TTATTGAAGA ACGTGGAGGC GGAC          534
```

(2) INFORMATION ZU SEQ ID NO: 7:

(i) SEQUENZ CHARAKTERISTIKA:
        (A) LÄNGE: 22 Basenpaare
        (B) ART: Nukleinsäure
        (C) STRANGFORM: Einzel
        (D) TOPOLOGIE: linear

(ii) ART DES MOLEKÜLS: DNS (genomisch)

(iii) HYPOTHETISCH: NEIN

(iii) ANTISENSE: NEIN

(vi) URSPRÜNLICHE HERKUNFT:
        (A) ORGANISMUS: Staphylococcus aureus

(xi) SEQUENZBESCHREIBUNG: SEQ ID NO: 7:

```
GGTGACTCAT CTATTTATGA AG                                              22
```

(2) INFORMATION ZU SEQ ID NO: 8:

(i) SEQUENZ CHARAKTERISTIKA:
        (A) LÄNGE: 22 Basenpaare
        (B) ART: Nukleinsäure
        (C) STRANGFORM: Einzel
        (D) TOPOLOGIE: linear

(ii) ART DES MOLEKÜLS: DNS (genomisch)

(iii) HYPOTHETISCH: NEIN

(iii) ANTISENSE: NEIN

(vi) URSPRÜNLICHE HERKUNFT:
        (A) ORGANISMUS: Staphylococcus aureus

(xi) SEQUENZBESCHREIBUNG: SEQ ID NO: 8:

```
GGTGACTCAT CTATCTATGA AG                                              22
```

(2) INFORMATION ZU SEQ ID NO: 9:

(i) SEQUENZ CHARAKTERISTIKA:
        (A) LÄNGE: 22 Basenpaare
        (B) ART: Nukleinsäure
        (C) STRANGFORM: Einzel
        (D) TOPOLOGIE: linear

(ii) ART DES MOLEKÜLS: DNS (genomisch)

(iii) HYPOTHETISCH: NEIN

(iii) ANTISENSE: NEIN

    (vi) URSPRÜNLICHE HERKUNFT:
        (A) ORGANISMUS: Staphylococcus aureus

    (xi) SEQUENZBESCHREIBUNG: SEQ ID NO: 9:

GGTGACTTAT CTATTTATGA AG               22

(2) INFORMATION ZU SEQ ID NO: 10:

    (i) SEQUENZ CHARAKTERISTIKA:
        (A) LÄNGE: 22 Basenpaare
        (B) ART: Nukleinsäure
        (C) STRANGFORM: Einzel
        (D) TOPOLOGIE: linear

    (ii) ART DES MOLEKÜLS: DNS (genomisch)

    (iii) HYPOTHETISCH: NEIN

    (iii) ANTISENSE: NEIN

    (vi) URSPRÜNLICHE HERKUNFT:
        (A) ORGANISMUS: Staphylococcus aureus

    (xi) SEQUENZBESCHREIBUNG: SEQ ID NO: 10:

GGTGACTTAT CTATCTATGA AG               22

(2) INFORMATION ZU SEQ ID NO: 11:

    (i) SEQUENZ CHARAKTERISTIKA:
        (A) LÄNGE: 22 Basenpaare
        (B) ART: Nukleinsäure
        (C) STRANGFORM: Einzel
        (D) TOPOLOGIE: linear

    (ii) ART DES MOLEKÜLS: DNS (genomisch)

    (iii) HYPOTHETISCH: NEIN

    (iii) ANTISENSE: NEIN

    (vi) URSPRÜNLICHE HERKUNFT:
        (A) ORGANISMUS: Staphylococcus aureus

    (xi) SEQUENZBESCHREIBUNG: SEQ ID NO: 11:

GGTGACTCAT CTATTTATAA AG               22

(2) INFORMATION ZU SEQ ID NO: 12:

    (i) SEQUENZ CHARAKTERISTIKA:
        (A) LÄNGE: 22 Basenpaare
        (B) ART: Nukleinsäure
        (C) STRANGFORM: Einzel
        (D) TOPOLOGIE: linear

    (ii) ART DES MOLEKÜLS: DNS (genomisch)

    (iii) HYPOTHETISCH: NEIN

    (iii) ANTISENSE: NEIN

    (vi) URSPRÜNLICHE HERKUNFT:
        (A) ORGANISMUS: Staphylococcus aureus

    (xi) SEQUENZBESCHREIBUNG: SEQ ID NO: 12:

GGTGATTCAT CTATTTATAA AG               22

(2) INFORMATION ZU SEQ ID NO: 13:

    (i) SEQUENZ CHARAKTERISTIKA:
        (A) LÄNGE: 22 Basenpaare
        (B) ART: Nukleinsäure

```
        (C)  STRANGFORM: Einzel
        (D)  TOPOLOGIE: linear

   (ii)  ART DES MOLEKÜLS: DNS (genomisch)

  (iii)  HYPOTHETISCH: NEIN

  (iii)  ANTISENSE: NEIN

   (vi)  URSPRÜNLICHE HERKUNFT:
        (A)  ORGANISMUS: Staphylococcus aureus


   (xi)  SEQUENZBESCHREIBUNG: SEQ ID NO: 13:
GGTGACGCAT CTATTTATGA AG                                          22
```